# FASCICULE DE BREVET EUROPEEN

(11) **EP 2 403 545 B1**
(45) Date de publication et mention de la délivrance du brevet: **17.04.2013**
(21) Numéro de dépôt: 10715298.5
(22) Date de dépôt: 05.03.2010
(51) Int. Cl.: A61L 27/16, A61L 27/48, A61L 27/50, A61L 24/00

(54) **CIMENT POUR COMBLEMENT OSSEUX**
ZEMENT ZUM AUFFÜLLEN VON KNOCHEN
CEMENT FOR FILLING BONES

(30) Priorité: 05.03.2009 FR 0951388
(43) Date de publication de la demande: 11.01.2012
(73) Titulaire: Teknimed, 65502 Vic-en-Bigorre Cedex (FR)
(72) Inventeur: LEONARD, Alain, F-65500 Caixon (FR); LAVERGNE, Claudine, F-65500 Caixon (FR); SENDER, Cyril, F-31400 Toulouse (FR); DONAZZON, Benoît, F-31570 Lanta (FR)
(74) Mandataire: Hartmann, Jean-Luc
(86) Numéro de dépôt international: PCT/FR2010/050375
(87) Numéro de publication internationale: WO 2010/100382

(56) Documents cités:
- WO-A-2008/109045
- KIM S B ET AL: "The characteristics of a hydroxyapatite-chitosan-PMMA bone cement" BIOMATERIALS, ELSEVIER SCIENCE PUBLISHERS BV., BARKING, GB, vol. 25, no. 26, 1 novembre 2004 (2004-11-01), pages 5715-5723, XP004508617 ISSN: 0142-9612
- BOESEL ET AL: "A review on the polymer properties of Hydrophilic, partially Degradable and Bioactive acrylic Cements (HDBC)" PROGRESS IN POLYMER SCIENCE, PERGAMON PRESS, OXFORD, GB, vol. 33, no. 2, 19 janvier 2008 (2008-01-19), pages 180-190, XP022427741 ISSN: 0079-6700

## Description

La présente invention se rapporte au domaine des ciments polymériques, en particulier des ciments acryliques, utilisés pour la réparation des traumatismes des os et des articulations.

Elle a pour objet un ciment fluide à usage médical ayant des propriétés mécaniques adaptées au comblement du tissu osseux spongieux, ainsi qu'une composition binaire destinée à la préparation d'un tel ciment.

Les ciments osseux sont utilisés depuis de nombreuses années pour favoriser la fixation des implants artificiels au squelette. Le ciment servant de jonction entre l'os et l'implant doit répondre à un certain nombre d'exigences, notamment être non toxique et biocompatible. Certains ciments ont même été étudiés pour leurs propriétés bioactives, c'est-à-dire pour leur action favorisant l'adhérence et la croissance cellulaire sur l'implant.

Depuis le milieu des années 80, l'emploi des ciments s'est étendu à la réparation osseuse, et en tout premier lieu à la vertébroplastie percutanée. Cette technique peu invasive, permet d'injecter un ciment à travers un trocart dans une vertèbre fracturée, pour assurer un volume osseux et une stabilisation. La première vertébroplastie percutanée a été réalisée en 1984 et a connu depuis un succès croissant, ouvrant la voie à la réparation plastique d'autres types d'os.

Aux Etats-Unis, il se produit annuellement entre 400 000 et 500 000 fractures vertébrales ostéoporétiques cliniques. Approximativement un tiers de ces patients développe une douleur chronique débilitante qui ne répond pas au traitement conservateur. Pour un grand nombre de personnes, ceci représente la fin d'un mode de vie indépendant. Ces patients peuvent être efficacement traités par l'injection percutanée de ciment osseux dans le corps vertébral fracturé. Dans cette technique, une pâte d'un ciment chirurgical est injectée avec précaution par voie percutanée, par l'intermédiaire d'une longue canule, directement dans l'os spongieux du corps vertébral fracturé. Le principal bénéfice de cette technique réside dans le fait que jusqu'à 90 % des patients ressentent un soulagement de la douleur en l'espace de 24 heures. (Jensen, M. E. et al. (1997). Am. J. Neuroradiol. 18, 1897-1904).

Les ciments utilisés jusqu'à présent sont des polymères organiques, formés à partir d'un mélange d'un prépolymère, généralement du PMMA (poly(méthacrylate de méthyle)) et d'un monomère, généralement du MMA (méthacrylate de méthyle), réagissant en présence d'un activateur de polymérisation.

La plupart des ciments disponibles dans le commerce sont présentés sous forme de deux composants séparés: une poudre comprenant principalement des billes de prépolymère, et un liquide contenant principalement le monomère. On incorpore en général l'initiateur, par exemple du peroxyde de benzoyle (BPO), à la poudre, tandis que le liquide contient un activateur chimique (catalyseur) tel que la diméthyl-para-toluidine (DMPT), la réaction de polymérisation démarrant lorsque les deux composants sont mélangés. Pour éviter une polymérisation spontanée pouvant se produire éventuellement durant le stockage, on incorpore en outre au composant liquide un stabilisant, communément l'hydroquinone. L'activateur et l'initiateur sont introduits à raison de 0,2 à 2,5 % en poids dans le composant correspondant, le stabilisant faisant quant à lui effet à quelques dizaines de ppm.

Afin de visualiser le ciment durant et après l'opération par des moyens radiologiques, une substance radio-opaque peut être ajoutée à la poudre de billes de prépolymère, le plus souvent du sulfate de baryum (BaSO₄) ou du dioxyde de zirconium (ZrO₂).

Ces compositions binaires pour la préparation de ciments osseux, conçues à l'origine pour la fixation d'implants et le scellement de prothèse, répondent à des critères mécaniques de résistance à la flexion et à la compression, de neutralité chimique et de biocompatibilité. Elles sont homologuées pour un usage médical et ont prouvé leurs qualités à long terme lorsque le squelette est soumis à des efforts importants et répétés. C'est pourquoi les ciments osseux pour la fixation d'implants ont été considérés comme des matériaux de prédilection pour la chirurgie de reconstruction des os et en particulier en vertébroplastie ou kyphoplastie.

Alors que cette technique est de plus en plus pratiquée, des inquiétudes apparaissent concernant les risques associés, et notamment les fractures des vertèbres adjacentes aux corps vertébraux cimentés. Il s'agit d'une des complications courantes les plus sérieuses, impliquant une nouvelle procédure de vertébroplastie. Outre le volume et la distribution du ciment injecté qui jouent des rôles importants dans le rétablissement des propriétés mécaniques des corps vertébraux fracturés (Liebschner M.A., et al. (2001) Spine 26, 1547-54), la forte rigidité des ciments par rapport à l'os vertébral trabéculaire est considérée comme un des facteurs principaux du risque de fracture des niveaux adjacents aux corps cimentés (Zoarski G.H., et al. (2002) J. Vasc. Interv. Radiol. 13, 139-148, Baroud G. et al. (2006) Joint Bone Spine 73, 144-150).

De la cimentation d'une vertèbre fracturée résulte une redistribution du champ de contraintes au sein de la vertèbre traitée et des corps vertébraux adjacents à l'origine de fractures ultérieures [Fribourg D., et al. (2004) Spine 29, 2270-76, Liebschner M.A., et al. (2001) Spine 26, 1547-54, Baroud G., et al. (2003) Comp. Methods Biomech. Biomed. Eng. 6, 133-39, Polikeit A., et al. (2003) Spine, 28(10), 991-96]. La présence d'une vertèbre cimentée dans une unité vertébrale fonctionnelle (deux corps vertébraux adjacents et un disque intervertébral) a pour effet de diminuer significativement sa résistance à la rupture de 19 % en moyenne, la fracture survenant systématiquement dans un corps vertébral non cimenté (Berlemann U., et al. (2002) J. Bone Joint Surg. Br. 84, 748-52).

Ce résultat appuie d'autres études biomécaniques qui ont montré que l'injection de ciments acryliques dans une vertèbre isolée et non fracturée augmente sa résistance et sa rigidité à la compression (Liebschner M.A., et al. (2001) Spine 26, 1547-54, Belkoff S.M., et al. (1999) Bone 25, 23S-26S, Wilson D.R., et al. (2000) Spine 25, 158-65, Belkoff S.M., et al. (2000) Spine 25, 1061-64, Heini P.F., et al. (2001) Eur. Spine J. 10, 164-71]. Des modélisations par éléments finis ont mis en évidence une augmentation de la rigidité en compression des corps vertébraux adjacents entre 13 et 18 % et de la pression hydrostatique au sein des disques intervertébraux d'environ 11 % après simulation d'un acte de vertébroplastie avec un ciment acrylique (Baroud G., et al. (2003) Comp. Methods Biomech. Biomed. Eng. 6, 133-39, Polikeit A., et al. (2003) Spine, 28(10), 991-96, Baroud G., et al. (2003) Eur. Spine J. 12, 421-26).

Une augmentation de la pression discale a été mise en évidence (Ananthakrishnan D., et al. (2003) Annual meeting of the American academy of orthopaedic surgeons, New Orleans, 472) et peut être expliquée par une déformation de la courbure et une diminution de la complaisance des plateaux vertébraux des vertèbres augmentées (Baroud G., et al. (2003) Comp. Methods Biomech. Biomed. Eng. 6, 133-39). Une étude biomécanique récente portant sur l'évolution des propriétés mécaniques d'unités vertébrales fonctionnelles constituées de trois vertèbres et deux disques a montré que les plateaux vertébraux des vertèbres cimentées fracturent systématiquement contrairement aux unités fonctionnelles de contrôle (Moore S., et al. (2008) Griboi 2008, Montréal, Canada, p 22).

Les ciments acryliques actuels admettent des propriétés mécaniques répondant aux exigences règlementaires en vigueur. Cependant, ces exigences ont été établies pour des ciments dont les indications spécifiques sont la fixation d'implants ou le scellement de prothèses, et pour lesquelles les critères en termes de propriétés mécaniques ne sont pas adaptés à la vertébroplastie, kyphoplastie ou cimentoplastie. Le module élastique et la résistance mécanique des ciments à base de résines acryliques, exigés par les textes normatifs, sont très élevés par rapport aux propriétés mécaniques de l'os spongieux humain. Cette différence d'impédance mécanique a été identifiée comme un facteur de risque aggravant la survenue de fractures des vertèbres adjacentes aux corps vertébraux cimentés.

La rigidité d'une structure peut être reliée à son module d'élasticité ou module de Young, valeur physique aisément déterminée par un homme du métier. Des mesures en compression statique sur de l'os spongieux vertébral humain ont permis de déterminer un module de Young compris entre 100 et 800 MPa, valeur dépendante de la densité osseuse, de l'orientation des trabécules, de la préparation des échantillons... alors que les ciments classiques de vertébroplastie présentent des valeurs comprises entre 1800 et 2500 MPa. Le module élastique et la résistance mécanique en compression de l'os spongieux vertébral humain sont respectivement 20 et 36 fois plus faibles en moyenne par rapport aux ciments acryliques actuellement injectés par vertébroplastie ou kyphoplastie (Hou F.J., et al. (1998) J. Biomech. 31, 1009-15, Fyhrie D.P. et al. (2000) Bone 26(2), 169-73, Banse X., et al. (2002) J. Bone & Mineral Res. 17(9), 1621-28, Shim V.P.W., et al. (2005) Int. J. Impact Eng. 32, 525-540).

Afin d'éliminer cette disparité mécanique, les propriétés mécaniques des ciments PMMA doivent être ajustées à celles de l'os spongieux vertébral. Il en résulterait alors une meilleure répartition des contraintes, et donc une réduction notable du risque de fractures adjacentes.

Les conditions de mise en oeuvre des ciments en chirurgie percutanée impliquent que soient respectées des critères d'injectabilité du ciment osseux, sous peine d'accidents dont les effets peuvent être dramatiques pour le patient, comme les paraplégies. C'est pourquoi le praticien doit disposer d'un ciment suffisamment fluide pour qu'il puisse s'écouler à travers un trocart de quelques millimètres de diamètre, et qu'il conserve cette fluidité suffisamment longtemps pour que le praticien ait le temps d'opérer en toute quiétude.

D'autre part, le ciment injecté, même en petites quantités, doit pouvoir être visualisé en permanence durant l'opération par fluoroscopie.

Un autre inconvénient sérieux réside dans le fait que la réaction de polymérisation des ciments osseux acryliques est exothermique, la température pouvant dépasser 80°C au coeur du ciment dans le corps vertébral. En effet, alors qu'en arthroplastie l'épaisseur de ciment acrylique réalisant la jonction entre l'os et la prothèse ne dépasse pas quelques millimètres favorisant l'évacuation de la chaleur produite par la réaction de polymérisation, en vertébroplastie elle peut être supérieure au centimètre. Cette configuration a pour conséquence de limiter l'évacuation des calories produites et ainsi contribuer à une élévation importante de la température au coeur du ciment. Cette température excessive entraîne une nécrose des tissus avoisinants. Une température ne dépassant pas 50 à 60°C est préférable pour l'injection de ciments chirurgicaux.

Ainsi, les ciments actuellement connus, s'ils sont performants pour la stabilisation de fractures de corps vertébraux ostéoporotiques par voie percutanée, ne prennent pas en compte les spécificités biomécaniques à l'origine du phénomène des fractures adjacentes.

L'art antérieur met en évidence l'existence d'une composition qui tente de répondre en partie à cette problématique. Il s'agit d'un mélange injectable comprenant un ciment osseux classique à deux composants poudre/liquide, un troisième composant constitué par un liquide hydrophile non miscible avec le ciment, et un agent de contraste aux rayons X de nature organique et de préférence sous forme de solution aqueuse pouvant remplacer entièrement ledit troisième composant liquide non miscible avec le ciment (EP1592463B1).

Le composant liquide non miscible est approprié pour partir au lavage dudit mélange aboutissant à une matière poreuse interconnectée de remplacement d'os. La porosité résultante a pour effet de diminuer la rigidité du mélange polymérisé à un niveau comparable à celui de l'os spongieux.

Le choix des auteurs de ne pas utiliser d'agent de contraste solide inorganique hydrophile comme le sulfate de baryum (BaSO₄) ou l'oxyde de zirconium (ZrO₂) s'explique par l'utilisation d'un troisième composant liquide aqueux et l'obtention d'une porosité interconnectée selon leur invention. En effet, une accumulation sélective de ces agents de contraste dans la phase aqueuse aboutirait, après lavage, au relargage des particules de BaSO₄ ou ZrO₂ dans l'organisme à un niveau de toxicité élevée. Ce phénomène est favorisé par l'interconnectivité des pores qui permet une évacuation aisée des particules présentes au sein du mélange injectable vers l'organisme via la libre circulation des fluides physiologiques. En termes de biocompatibilité, la diminution de la rigidité du mélange polymérisé par la création d'une porosité ouverte et interconnectée à l'aide d'un troisième composant liquide hydrophile ne peut donc pas être conçue en présence d'un agent de contraste solide pulvérulent. C'est pour cela qu'il est revendiqué d'utiliser un agent de contraste organique en solution aqueuse à base d'iode et à hauteur de 20 % en poids du mélange injectable. Néanmoins, l'homme du métier peut aisément reconnaître que la teneur maximale en agent de contraste iodé décrit dans le brevet EP1592463B1 ne permet pas d'obtenir un contraste aussi élevé que par l'utilisation d'agents de contraste solides tels que le BaSO₄ ou le ZrO₂ pouvant être utilisés actuellement jusqu'à 60 % en poids des ciments chirurgicaux en vertébroplastie ou kyphoplastie.

D'autre part, pour que le ciment polymérisé soit toujours radio-opaque après lavage de l'agent de contraste contenu dans le composant liquide non miscible au ciment, il est mentionné qu'un agent de contraste lipophile miscible dans la phase PMMA peut être ajouté. L'ajout d'un quatrième composant dans cette formulation ne rend sa préparation que plus délicate.

Ainsi, certaines exigences liées à l'utilisation d'un ciment acrylique pour le comblement de tissus osseux en termes de visualisation par fluoroscopie du ciment durant l'acte chirurgical et lors du suivi des patients, de facilité de préparation, et d'efficacité de la troisième phase introduite dans le ciment à promouvoir la création d'une structure poreuse contrôlée (contrôle de la taille et de la dispersion des pores formés) ne sont pas pleinement résolues par l'invention du brevet EP1592463B1.

L'objet de la présente invention est un ciment osseux adapté à un usage en chirurgie de reconstruction de l'os, en particulier pour le comblement d'un corps vertébral, permettant de répondre aux exigences précitées demeurant non satisfaites par la création d'une structure composite de substitution osseuse, composée d'une matrice de composition identique à un ciment osseux radio-opaque et d'une dispersion homogène de zones de densité inférieure et de dimensions contrôlées. Cette structure composite admet une rigidité équivalente ou légèrement supérieure à celle de l'os spongieux vertébral humain.

Le cahier des charges du ciment osseux selon l'invention est le suivant:
- Injectabilité,
- Temps de prise supérieur ou égal à 15 minutes,
- Opacité en fluoroscopie pendant et après injection,
- Faible exothermicité (température de polymérisation inférieure à 60°C),
- Module de Young en compression inférieur à 1500 MPa.

L'expression « temps de prise » fait référence au temps défini selon la norme ISO5833, Annexe C.

Un tel ciment doit être compatible avec un usage médical du point de vue de sa toxicité et de sa biocompatibilité.

Dans la présente invention, il a été trouvé qu'il était possible de formuler un ciment osseux à rigidité équivalente ou légèrement supérieure à celle de l'os spongieux vertébral humain à partir d'un ciment acrylique à base de polyméthylméthacrylate et de méthylméthacrylate monomère répondant au cahier des charges ci-dessus, en introduisant audit ciment acrylique une dispersion homogène de zones de dimensions contrôlées et de densité inférieure au ciment acrylique, tout en conservant les qualités exigées en termes de biocompatibilité, d'injectabilité, de temps de prise, d'exothermicité, de radio-opacité en chirurgie interventionnelle.

Dans le ciment de la présente invention, les zones de dimensions contrôlées et de densité inférieure au ciment acrylique sont constituées par des particules solides souples hydrophiles et calibrées de gélatine, de poly(glycérol sébacate) ou un mélange des deux. La gélatine est une substance d'origine animale entrant dans la composition de dispositifs médicaux présents sur le marché, le poly(glycérol sébacate), ou PGS, est un élastomère synthétique, biocompatible, biorésorbable et hydrophile ayant des propriétés mécaniques similaires aux dispositifs médicaux contenant de la gélatine tel que l'obturateur diaphysaire CEMSTOP^{□}. Le PGS est un élastomère souple à la température physiologique humaine. Les particules de gélatine ou de PGS présentent l'avantage de diminuer le module d'élasticité des ciments sans modifier la facilité d'injection et la radio-opacité caractéristiques des ciments acryliques en vertébroplastie. En effet, les particules de gélatine et/ou de PGS dispersées dans le ciment permettent de créer un matériau composite flexible, dont les propriétés mécaniques imitent celles de l'os spongieux vertébral en opposition aux produits actuellement sur le marché qui admettent des propriétés mécaniques beaucoup plus élevées. La biocompatibilité connue des ciments d'une part, et de la gélatine ou du PGS d'autre part, confère à leur mélange une biocompatibilité assurée.

Plus précisément, la présente invention a pour objet un ciment osseux injectable pour le comblement osseux aux propriétés mécaniques équivalentes à celles de l'os spongieux vertébral comprenant 70 à 99 % en poids d'un polymère acrylique associé à un composé radio-opaque de nature inorganique et de 1 à 30 % en poids de particules solides souples hydrophiles et calibrées capables d'absorber les liquides physiologiques sans créer de porosité interconnectée. Le ciment osseux selon la présente invention est de grade médical et trouve des applications particulièrement avantageuses en vertébroplastie, kyphoplastie ou cimentoplastie.

Les particules solides souples hydrophiles de gélatine et/ou de PGS sont ajoutées dans des proportions allant de 1 à 30 % en poids total de ciment. De préférence, on choisira une quantité comprise entre 10 et 20 % en poids total de ciment.

Des particules solides souples hydrophiles convenant particulièrement à l'élaboration du ciment osseux selon la présente invention sont de forme globalement sphériques, de diamètre moyen compris entre 50 et 1000 µm. De préférence, le diamètre moyen des particules est supérieur à 300 µm En effet, le demandeur de la présente invention a mis en évidence que jusqu'à 300 µm, plus les dimensions des particules solides souples hydrophiles sont faibles (plus la surface spécifique est élevée), plus la quantité de monomère liquide adsorbée par ces dernières lors de la préparation du ciment est importante, plus la quantité de monomère disponible pour la dissolution partielle des billes de prépolymère de polyméthylméthacrylate est faible et plus l'obtention d'un ciment fluide est difficile. Cette difficulté pourrait être levée par un apport plus important de monomère liquide mais ceci augmenterait le taux résiduel de monomère dans le ciment durci, donc le taux de relargage de monomère dans l'organisme et par conséquent le niveau de toxicité du ciment. Des particules hydrophiles de forme aciculaire ou en paillettes sont également objets de la présente invention.

De manière inattendue, il a été trouvé que les particules solides souples hydrophiles calibrées remplissent une autre fonction particulièrement intéressante dans le ciment selon l'invention. En effet, il a été observé que la présence de particules solides hydrophiles calibrées provoque une diminution de la température maximale atteinte durant la polymérisation du ciment. Cet effet est d'autant plus important que les dimensions des particules solides hydrophiles sont faibles et que leur quantité en poids total de ciment est élevée. Sans entrer dans des études théoriques, on suppose que les particules solides hydrophiles calibrées agissent comme des dissipateurs de chaleur. Le résultat de cette diminution de température de polymérisation, qui peut être de quelques dizaines de degrés Celsius par rapport aux ciments acryliques actuellement connus, est une réduction voire une absence de nécrose des tissus biologiques au contact avec le ciment.

Le demandeur a montré que la présence de particules solides souples hydrophiles calibrées dispersées de façon homogène dans le ciment acrylique diminue progressivement la rigidité du mélange durci selon l'invention. La diffusion progressive des liquides physiologiques au sein du ciment acrylique polymérisé et leur absorption consécutive par les particules solides souples hydrophiles provoque une diminution progressive et contrôlée, suivie d'une stabilisation de sa rigidité à un niveau identique ou légèrement supérieur à celui de l'os spongieux vertébral dans lequel il est injecté. L'évolution temporelle des propriétés mécaniques du mélange selon la présente invention est liée à la diminution de la densité des zones occupées par les particules solides ayant absorbées les liquides physiologiques, et non pas à la création progressive d'une porosité interconnectée.

Il en résulte une porosité de type ouverte et non interconnectée au sein du ciment selon l'invention, c'est à dire qui comprend des pores isolés les uns des autres constitués par les particules solides souples hydrophiles, dont certains émergent à la surface et sont « lavés » par les fluides physiologiques qui entrent au contact de la surface du ciment, créant une porosité ouverte. Ces pores abritant les particules solides souples hydrophiles ne sont pas reliés les uns aux autres à l'intérieur du ciment selon l'invention, ils ne forment pas de microréseau de canaux interconnectés ce qui évite de fragiliser l'ensemble du ciment et permet d'obtenir des propriétés mécaniques plus proches de celles de l'os spongieux.

Le brevet EP1592463B1 décrit quant à lui une porosité ouverte et interconnectée dans laquelle les pores sont reliés les uns aux autres en un microréseau de canaux internes et émergent à la surface, aboutissant à une structure de substitution osseuse dont la rigidité est immédiatement stable dans le temps après injection du ciment et lavage du composant fluide. L'utilisation de particules solides souples hydrophiles décrite selon la présente invention permet de diminuer la rigidité des ciments acryliques de façon progressive et contrôlée. La rigidité du ciment juste après son injection est identique aux ciments osseux actuellement connus en vertébroplastie, assurant une stabilisation de la fracture et un effet antalgique immédiat. La rigidité finale stabilisée, est identique à celle de l'os spongieux vertébral humain et contribue à la diminution du risque de fracture des vertèbres adjacentes aux vertèbres cimentées.

Certains auteurs ont décrit des ciments à base de polymère de méthacrylate comprenant une hydroxyapatite associés à une poudre de chitosane dans le but d'améliorer les propriétés de biodégradabilité et de résistance mécanique du ciment (Seok Bong Kim et al. « The characteristics of a hydroxyapatite-chitosan-PMMA bone cement », 1er novembre 2004). Là encore, la porosité obtenue est de type ouverte et interconnectée, avec les inconvénients de fragilité associés à de telles structures internes mentionnés ci-dessus, mais également en terme de risque de relargage de monomère MMA non réagis et de particules solides. Les pores formés dans ce type de ciment sont vides après dissolution du chitosane par les fluides physiologiques contrairement à la présente invention où les particules solides souples hydrophiles restent piégées à l'intérieur du ciment, contribuant à une meilleure résistance du ciment qui se rapproche davantage de celle d'un os spongieux.

Un autre avantage de la présente invention est de conférer aux ciments chirurgicaux une rigidité équivalente à celle de l'os spongieux vertébral par l'utilisation d'une fraction volumique plus faible en particules souples hydrophiles calibrées en comparaison au troisième composant liquide revendiqué dans le brevet EP1592463B1. En effet, à taux volumique identique les particules de gélatine diminuent davantage la rigidité des ciments acryliques sans créer de porosité interconnectée, qui par ailleurs augmente la surface spécifique des implants acryliques et par conséquent les risques de génération et de diffusion de débris issus de l'implant ou de relargage de composants du ciment en quantité toxique dans l'organisme.

Bien que la biocompatibilité ou la reconnaissance des ciments acryliques par les cellules osseuses aient été améliorées par l'introduction dans les formulations de particules ostéoconductrice telle que des phosphates de calcium, une biointégration mécanique n'a jamais été observée. Une caractéristique avantageuse de l'invention est la création d'une porosité à la surface du ciment au contact avec l'os ou porosité ouverte. Cette porosité surfacique due à la biodégradation des particules solides souples hydrophiles calibrées présentes à la surface du ciment selon l'invention et entrant en contact avec les fluides physiologiques et les tissus calcifiés permettrait une repousse osseuse dans les pores ainsi créés et assurerait la biointégration mécanique du ciment au sein du corps vertébral.

Le polymère acrylique selon l'invention est composé d'au moins un prépolymère de polyméthylméthacrylate (ou PMMA) et d'au moins un méthylméthacrylate monomère (ou MMA) communément utilisés pour la préparation de ciments acryliques. Des copolymères à base de methylmethacrylate - styrene peuvent également entrer dans le cadre de la présente invention. Les poudres de prépolymère se présentent sous la forme de billes. La masse molaire de ces poudres est comprise entre 150 000 et 1 500 000 g/mole. Le diamètre moyen des particules est compris entre 30 µm et 150 µm. Le monomère est l'ester méthylique de l'acide méthacrylique. Les monomères et prépolymères comme le MMA, le PMMA et les copolymères MMA-styrene à usage médical sont disponibles dans le commerce.

Selon une caractéristique préférée de la présente demande, le composé radio-opaque inorganique est présent dans le ciment acrylique et donc présent durant toute la vie de l'implant. Il permet de suivre visuellement l'injection du ciment pour éviter tout risque de fuite extra-osseuse, et donne la possibilité de réaliser un suivi médical post-opératoire. Le composé radio-opaque peut être choisi parmi les composés connus et compatibles avec un usage médical. Il est de préférence choisi dans le groupe composé du sulfate de baryum et du dioxyde de zirconium. Le sulfate de baryum (BaSO₄) est un radio-opacifiant communément utilisé dans les ciments pour la fixation d'implants, dont l'innocuité est reconnue. Il se présente en général sous forme poudre dont les particules ont un diamètre moyen de 1 à 10 □m. Le dioxyde de zirconium (ZrO₂) peut être utilisé en variante. Il est introduit sous forme de poudre dont les particules ont un diamètre moyen de 20 µm.

Pour des applications en vertébroplastie ou kyphoplastie, la composition radio-opaque représente une fraction importante du ciment. Elle a pour but d'injecter le ciment sous contrôle continu par fluoroscopie. Elle peut être constituée d'un composé radio-opaque pur ou en mélange avec d'autres ingrédients. On peut avantageusement utiliser un phosphate de calcium, en particulier une hydroxyapatite phosphocalcique de formule Ca₁₀(PO₄)₆(OH)₂. L'introduction de phosphate de calcium dans la composition apporte un effet doublement bénéfique, d'une part en améliorant l'homogénéité du ciment et par suite sa malléabilité, et d'autre part en augmentant sa biocompatibilité. En effet, il est connu que l'hydroxyapatite favorise la repousse osseuse en stimulant l'activité biologique des ostéoblastes et leur prolifération. Elle a été étudiée à ce titre, sans que ses propriétés mécaniques soient d'ailleurs mises à profit. De manière alternative, l'hydroxyapatite peut être remplacée dans la présente invention par un phosphate tricalcique (TCP). De manière avantageuse, la composition radio-opaque présente dans le ciment selon l'invention comprend un composé radio-opaque et du phosphate de calcium.

Le ciment selon l'invention peut enfin contenir un certain nombre de réactifs favorisant le contrôle de la polymérisation. Notamment, il peut comprendre, outre les ingrédients mentionnés ci-dessus, une quantité efficace d'un ou plusieurs des réactifs suivants : un activateur chimique de polymérisation, un initiateur de polymérisation, un stabilisant. L'homme du métier connaît ces réactifs et maîtrise leur mise en oeuvre.

Un initiateur de réaction peut avantageusement être choisi parmi les catalyseurs de polymérisation tels que le peroxyde de benzoyle (BPO). L'activateur ou accélérateur de la réaction de polymérisation est de préférence la N,N-diméthyl-para-toluidine (DMPT). Le stabilisant, de préférence l'hydroquinone, peut être ajouté pour éviter la polymérisation prématurée du monomère du fait de l'exposition à la chaleur ou à la lumière. Ces réactifs sont efficaces à des concentrations très faibles, que l'homme de l'art sait ajuster en fonction de la cinétique souhaitée.

L'introduction de particules solides souples hydrophiles calibrées dans le ciment a des répercussions sur ses caractéristiques physiques et chimiques, notamment son injectabilité, sa cinétique de polymérisation, ainsi que ses propriétés mécaniques. Le ciment osseux selon l'invention présente notamment un module de Young inférieur à 1 500 MPa. Pour obtenir des propriétés fonctionnelles optimales, les proportions d'ingrédients telles que définies par la présente demande doivent être respectées.

La présente invention ainsi décrite convient à une utilisation médicale dans le cadre de comblement osseux dans diverses parties du corps humain. Elle trouve cependant une application particulièrement avantageuse, du fait de ses propriétés mécaniques, dans le cas de vertébroplasties ou kyphoplasties percutanées où le ciment est injecté à travers un trocart dans un corps vertébral fracturé.

Le ciment selon l'invention présente une bonne fluidité dans les minutes suivant la mise en présence des ingrédients et peut être travaillé jusqu'à 15 minutes voire plus après sa préparation. La réaction de polymérisation entre le polyméthylméthacrylate et le monomère provoque la prise en masse du ciment. La température dans le corps vertébral durant la polymérisation est inférieure à 60°C. Dans la présente demande, le terme "ciment" ou "ciment fluide" correspond au ciment tel qu'il se présente après mélange des ingrédients. La composition du ciment sera considérée comme celle du ciment fluide prêt à l'emploi, avant solidification.

Un ciment osseux selon l'invention peut être obtenu par la préparation d'une composition binaire résultant du mélange d'une phase en poudre P comprenant principalement le polyméthylméthacrylate avec une phase liquide L comprenant principalement le méthylméthacrylate monomère dans un rapport P/L compris entre 3 et 4,6. De préférence, P/L est compris entre 3,4 et 4.

Les particules solides souples calibrées sont indifféremment incorporées dans l'une ou l'autre des deux phases P ou L. De préférence, elles sont incorporées dans la phase en poudre P.

La poudre de prépolymère de polyméthylméthacrylate, le méthylméthacrylate monomère et la composition radio-opaque d'une part, et les particules solides souples hydrophiles d'autre part, sont avantageusement apportés dans un rapport pondéral compris entre 2 et 100. De préférence ce rapport est compris entre 4 et 9. Ainsi, il a été déterminé que la formulation optimale est celle où la proportion de ciment est moindre et pour laquelle les propriétés mécaniques sont les plus faibles, en adéquation avec celles de l'os spongieux vertébral.

La composition binaire selon l'invention comprend de préférence une quantité efficace d'un ou plusieurs des réactifs suivants :
- dans le composant liquide L, un activateur chimique de polymérisation et un stabilisant;
- dans le composant en poudre P, un initiateur de polymérisation.

Par exemple, le composant liquide L peut comprendre de 0,7 % à 2,5 % de DMPT et 20 ppm d'hydroquinone. Le composant en poudre P peut comprendre de 0,2 % à 2 % de peroxyde de benzoyle. De préférence, le composant liquide L contient 1 % de DMPT, tandis que le composant en poudre P contient entre 0,3 % et 0,35 % de peroxyde de benzoyle.

Lors de l'utilisation en bloc opératoire, les deux composants, en poudre et liquide, sont mélangés. A cet instant, la phase poudre se dissout partiellement dans la phase liquide, donnant ainsi un mélange qui doit être suffisamment fluide pour pouvoir être injecté dans un corps vertébral. Au cours du mélange, l'activateur et l'initiateur réagissent pour produire des radicaux libres. Ces radicaux initient la réaction de polymérisation conduisant au durcissement progressif du ciment, selon une cinétique souhaitée.

Le ciment selon l'invention, une fois prêt, va réagir pour former une masse solide dans un laps de temps relativement court (de quelques minutes à quelques dizaines de minutes), la formulation ici revendiquée faisant prise au plus tôt en 15 minutes. Il est évident que les ingrédients réagissant entre eux doivent être mélangés uniquement au moment de l'emploi. C'est pourquoi il est commode de disposer d'une composition binaire constituée de deux prémélanges d'ingrédients qu'il suffit de réunir pour préparer le ciment selon l'invention. Ces prémélanges, l'un sous forme de poudre P contenant principalement le prépolymère de polyméthylméthacrylate, l'autre sous forme liquide L contenant principalement le méthylméthacrylate monomère, constituent les deux composants de ladite composition binaire.

Le dispositif selon l'invention peut être avantageusement utilisé pour la préparation d'un ciment fluide à usage médical pour le comblement de corps vertébraux.

Selon une variante avantageuse, le composant en poudre P comprend de 1 % à 36 % en particules souples, de préférence entre 13 % et 25 % en poids ramené au poids de poudre.

Les exemples suivant permettront de mieux comprendre l'invention, sans toutefois en limiter la portée.

Les abréviations suivantes sont utilisées :
PMMA:
   polyméthacrylate de méthyle
MMA:
   méthacrylate de méthyle
BPO:
   péroxyde de benzoyle
BaSO₄:
   sulfate de baryum
ZrO₂:
   dioxyde de zirconium
HAP:
   hydroxyapatite phosphocalcique
DMTP :
   diméthyl-para-toluidine
HQ:
   hydroquinone
P/L :
   rapport phase poudre / phase liquide, en poids.

### EXEMPLE 1 : Composition binaire à 13 % en poids de gélatine ramené au poids de poudre (10 % en poids de gélatine, ramené au poids du ciment)

### Phase Poudre (% en poids)

| | |
|---|---|
| PMMA | 43 |
| BPO | 0,4 |
| ZrO₂ | 39,2 |
| HAP | 4,4 |
| Gélatine | 13 |

### Phase liquide (% en poids)

| | |
|---|---|
| MMA | 99 |
| DMPT | 1 |
| HQ | 20 ppm |

avec P/L = 3,4
Module de Young final = 1000 MPa

### EXEMPLE 2 : Composition binaire à 25 % en poids de gélatine ramené au poids de poudre, (20 % en poids de gélatine, ramené au poids du ciment)

### Phase Poudre (% en poids)

| | |
|---|---|
| PMMA | 37,1 |
| BPO | 0,4 |
| ZrO₂ | 33,7 |
| HAP | 3,8 |
| Gélatine | 25 |

### Phase liquide (% en poids)

| | |
|---|---|
| MMA | 99 |
| DMPT | 1 |
| HQ | 20 ppm |

avec P/L = 4
Module de Young final = 500 MPa

### EXEMPLE 3 : Procédé de préparation d'un ciment osseux à rigidité adaptée

### • Composant en poudre :

La phase poudre est obtenue par mélange des divers ingrédients.

### • Composant liquide :

La phase liquide est préparée par dissolution de l'hydroquinone dans le monomère méthylméthacrylate. L'agitation est maintenue jusqu'à dissolution complète. Le DMPT est ensuite ajouté.

Les deux phases sont conditionnées séparément dans des récipients adaptés à leur conservation. Les kits de préparation instantanée comprennent un récipient contenant la phase liquide et un récipient contenant la phase poudre.

### • Composition binaire

Lors de l'utilisation en bloc opératoire, les récipients sont ouverts et leur contenu est mélangé. La poudre se dissout rapidement dans la phase liquide donnant un mélange fluide qui est injecté dans le corps vertébral du patient à travers une tubulure adéquate. L'initiateur BPO et l'activateur DMPT réagissent pour former des radicaux libres qui initient la réaction de polymérisation du ciment. Le chirurgien dispose alors d'au moins une quinzaine de minutes pour opérer, en contrôlant la procédure en permanence par fluoroscopie.

### EXEMPLE 4 : Tests selon Norme ISO 5833

La norme ISO 5833, intitulée « Implants chirurgicaux, ciments à base de résine acrylique » définit les caractéristiques requises par la réglementation et les tests standards permettant de quantifier ces caractéristiques. Les compositions décrites dans les exemples 1 et 2 précédents ont été testées pour déterminer leur temps de prise et la température maximale atteinte durant la polymérisation.

Les résultats obtenus pour les deux compositions des exemples 1 et 2 ainsi que la composition témoin, sans gélatine, sont présentés dans le tableau 1.

Tous les modes opératoires sont décrits en détail dans la norme ISO 5833.

| Tableau 1 | | |
|---|---|---|
| Composition | Temps de prise (mn) | Température maximale (°C) |
| Exemple 1 | 20,26 ± 0,44 | 52,0 ± 3,2 |
| Exemple 2 | 19,96 ± 0,43 | 47,2 ± 2,0 |
| Témoin | 18,24 ± 0,24 | 69,3 ± 2,1 |

On note que leurs caractéristiques répondent au cahier des charges défini précédemment pour des ciments osseux en vertébroplastie percutanée (temps de prise supérieur à 15 minutes, opacité en fluoroscopie).

### EXEMPLE 5 : Propriétés mécaniques

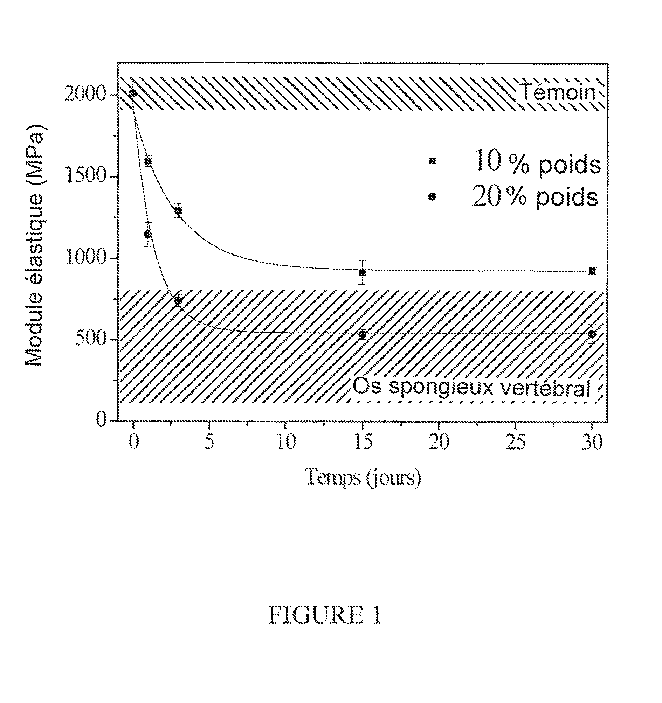

Une deuxième série d'essais concerne les propriétés mécaniques du ciment installé, c'est-à-dire son module de Young en compression. Ce paramètre a été déterminé à des temps réguliers (4, 24 heures, 3, 8 et 14 jours) sur des éprouvettes mises en forme selon la norme ISO 5833 et placées dans de l'eau distillée à 37°C sous faible agitation. Les résultats obtenus avec les compositions citées en exemple 1 et 2, et le ciment témoin, sans gélatine, sont présentés sur la Figure 1. Pour comparaison, on rappelle que le module de Young en compression de l'os spongieux vertébral humain est compris entre 100 et 800 MPa.

## Revendications

1. Ciment osseux injectable pour le comblement osseux aux propriétés mécaniques équivalentes à celles de l'os spongieux vertébral, ***caractérisé en ce* qu'**il comprend : 70 à 99 % en poids d'un polymère acrylique associé à un composé radio-opaque de nature inorganique et 1 à 30 % en poids de particules solides souples hydrophiles calibrées capables d'absorber les liquides physiologiques sans créer de porosité interconnnectée.

2. Ciment selon la revendication précédente, ***caractérisé en ce que*** les particules solides souples hydrophiles calibrées sont choisies parmi la gélatine, le poly(glycérol sébacate) ou un mélange des deux.

3. Ciment selon la revendication 1 ou 2, ***caractérisé en ce que*** les particules solides souples hydrophiles calibrées ont un diamètre moyen compris entre 50 et 1 000 µm.

4. Ciment selon la revendication précédente, ***caractérisé en ce que*** les particules solides souples hydrophiles calibrées ont un diamètre moyen compris entre 300 et 1 000 µm.

5. Ciment selon l'une quelconque des revendications précédentes, *caractérisé en ce qu*'il comprend 10 à 20 % en poids de particules solides souples hydrophiles calibrées.

6. Ciment selon l'une quelconque des revendications précédentes, ***caractérisé en ce que*** le polymère acrylique comprend au moins un prépolymère de polyméthylméthacrylate et au moins un méthylméthacrylate monomère.

7. Ciment selon l'une quelconque des revendications précédentes, ***caractérisé en ce que*** le composé radio-opaque inorganique est le sulfate de baryum ou le dioxyde de zirconium.

8. Ciment selon l'une quelconque des revendications précédentes, ***caractérisé en ce que*** le composé radio-opaque inorganique est associé à un phosphate de calcium.

9. Ciment selon l'une quelconque des revendications précédentes, ***caractérisé en ce* qu'**il comprend un ou plusieurs des réactifs suivants : un activateur chimique de polymérisation, de préférence la diméthyl-para-toluidime, un initiateur de polymérisation, de préférence le peroxyde de benzoyle, un stabilisant, de préférence l'hydroquinone.

10. Ciment selon l'une quelconque des revendications précédentes, ***caractérisé en ce* qu'**il présente un module de Young inférieur à 1 500 MPa.

11. Ciment selon l'une quelconque des revendications précédentes, obtenu à partir d'une composition binaire comprenant une phase en poudre P comprenant un polyméthylméthacrylate et une phase liquide L comprenant un méthylméthacrylate, ***caractérisée en ce que*** le rapport P/L est compris entre 3 et 4,6.

12. Ciment selon la revendication précédente, ***caractérisé en ce que*** le rapport P/L est de préférence compris entre 3,4 et 4.

13. Ciment selon la revendication 11, ***caractérisé en ce que*** les particules solides souples hydrophiles calibrées sont comprises dans la phase en poudre P.

14. Ciment selon l'une quelconque des revendications 11 à 13, ***caractérisé en ce que*** la poudre de prépolymère de polyméthylméthacrylate, le méthylméthacrylate monomère et la composition radio-opaque d'une part, et les particules solides souples hydrophiles d'autre part, sont apportés dans un rapport pondéral compris entre 2 et 100.

15. Ciment selon la revendication précédente, ***caractérisé en ce que*** la poudre de prépolymère de polyméthylméthacrylate, le méthylméthacrylate monomère et la composition radio-opaque d'une part, et les particules solides souples hydrophiles d'autre part, sont apportés dans un rapport pondéral compris entre 4 et 9.

## Claims

1. Injectable bone cement for filling bones with mechanical properties equivalent to those of vertebral spongy bone, wherein it comprises: 70 to 99% by weight of an acrylic polymer combined with an inorganic type radiopaque compound and 1 to 30% by weight of calibrated hydrophilic flexible solid particles able to absorb the physiological liquids without creating an interconnected porosity.

2. Cement according to the preceding claim, wherein the calibrated hydrophilic flexible solid particles are chosen from gelatin, poly(glycerol sebacate) or a mixture thereof.

3. Cement according to claim 1 or 2, wherein the calibrated hydrophilic flexible solid particles have an average diameter between 50 and 1,000 µm

4. Cement according to the preceding claim, wherein the calibrated hydrophilic flexible solid particles have an average diameter between 300 and 1,000 µm.

5. Cement according to any of the preceding claims, wherein it comprises 10 to 20% by weight of calibrated hydrophilic flexible solid particles.

6. Cement according to any of the preceding claims, wherein the acrylic polymer comprises at least one poly(methyl methacrylate) prepolymer and at least one methyl methacrylate monomer.

7. Cement according to any of the preceding claims, wherein the inorganic radiopaque compound is barium sulfate or zirconium dioxide.

8. Cement according to any of the preceding claims, wherein the inorganic radiopaque compound is combined with a calcium phosphate.

9. Cement according to any of the preceding claims, wherein it comprises one or more of the following reagents: a chemical polymerization activator, preferably dimethyl-para-toluidine, a polymerization initiator, preferably benzoyl peroxide, a stabilizer, preferably hydroquinone.

10. Cement according to any of the preceding claims, wherein it has a Young's modulus less than 1,500 MPa.

11. Cement according to any of the preceding claims, obtained from a binary composition, comprising a powder phase P comprising a poly(methyl methacrylate) and a liquid phase L comprising a methyl methacrylate, wherein the P/L ratio is between 3 and 4.6.

12. Cement according to the preceding claim, wherein the P/L ratio is preferably between 3.4 and 4.

13. Cement according to claim 11, wherein the calibrated hydrophilic flexible solid particles are included in the powder phase P.

14. Cement according to any of the preceding claims, wherein the poly(methyl methacrylate) prepolymer powder, the methyl methacrylate monomer and the radiopaque composition on the one hand, and the hydrophilic flexible solid particles on the other hand, are provided in a weight ratio between 2 and 100.

15. Cement according to the preceding claim, wherein the poly(methyl methacrylate) prepolymer powder, the methyl methacrylate monomer and the radiopaque composition on the one hand, and the hydrophilic flexible solid particles on the other hand, are provided in a weight ratio between 4 and 9.

## Patentansprüche

1. Injizierbarer Knochenzement zum Auffüllen von Knochen, der mechanische Eigenschaften aufweist, die mit denen von Spongiosa-Wirbelknochen äquivalent sind,
**dadurch gekennzeichnet,**
**dass** er enthält: 70 bis 99 Gew.-% eines Acrylpolymers, dem eine strahlenundurchlässige Verbindung anorganischer Art zugesetzt ist, und 1 bis 30 Gew.-% nachgiebige, hydrophile, klassierte Feststoffteilchen, die physiologische Flüssigkeiten absorbieren können, ohne dabei durchgängige Porenräume zu schaffen.

2. Zement nach dem vorangehenden Anspruch,
**dadurch gekennzeichnet,**
**dass** die nachgiebigen, hydrophilen, klassierten Feststoffteilchen ausgewählt sind aus Gelatine, Poly-Glyzerin-Sebacat oder aus einem Gemisch von diesen beiden.

3. Zement nach Anspruch 1 oder 2,
**dadurch gekennzeichnet,**
**dass** die nachgiebigen, hydrophilen, klassierten Feststoffteilchen einen mittleren Durchmesser von zwischen 50 und 1.000 µm haben.

4. Zement nach dem vorangehenden Anspruch,
**dadurch gekennzeichnet,**
**dass** die nachgiebigen, hydrophilen, klassierten Feststoffteilchen einen mittleren Durchmesser von zwischen 300 und 1.000 µm haben.

5. Zement nach einem der vorangehenden Ansprüche,
**dadurch gekennzeichnet,**
**dass** er 10 bis 20 Gew.-% nachgiebige, hydrophile, klassierte Feststoffteilchen aufweist.

6. Zement nach einem der vorangehenden Ansprüche,
**dadurch gekennzeichnet,**
**dass** das Acrylpolymer zumindest ein Polymethylmethacrylat-Vorpolymer und zumindest ein Methylmethacrylat-Monomer aufweist.

7. Zement nach einem der vorangehenden Ansprüche,
**dadurch gekennzeichnet,**
**dass** die strahlenundurchlässige, anorganische Verbindung Bariumsulfat oder Zirkoniumdioxid ist.

8. Zement nach einem der vorangehenden Ansprüche,
**dadurch gekennzeichnet,**
**dass** der strahlenundurchlässigen, anorganischen Verbindung Calciumphosphat zugesetzt ist.

9. Zement nach einem der vorangehenden Ansprüche,
**dadurch gekennzeichnet,**
**dass** es eines oder mehrere der nachfolgenden Reaktionsmittel aufweist: einen chemischen Polymerisationsaktivator, vorzugsweise Dimethyl-paratoluidin, einen Polymerisationsinitiator, vorzugsweise Benzoylperoxid, einen Stabilisator, vorzugsweise Hydrochinon.

10. Zement nach einem der vorangehenden Ansprüche,
**dadurch gekennzeichnet,**
**dass** er einen Elastizitätsmodul von unter 1.500 MPa aufweist.

11. Zement nach einem der vorangehenden Ansprüche, gewonnen aus einer binären Zusammensetzung, enthaltend eine Pulverphase P mit einem Polymethylmethacrylat und einer Flüssigphase L mit einem Methylmethacrylat,
**dadurch gekennzeichnet,**
**dass** das Verhältnis P/L zwischen 3 und 4,6 liegt.

12. Zement nach dem vorangehenden Anspruch,
**dadurch gekennzeichnet,**
**dass** das Verhältnis P/L vorzugsweise zwischen 3,4 und 4 liegt.

13. Zement nach Anspruch 11,
**dadurch gekennzeichnet,**
**dass** die nachgiebigen, hydrophilen, klassierten Feststoffteilchen in der Pulverphase P enthalten sind.

14. Zement nach einem der Ansprüche 11 bis 13,
**dadurch gekennzeichnet,**
**dass** das Pulver aus Polymethylmethacrylat-Vorpolymer, das Methylmethacrylat-Monomer und die strahlenundurchlässige Zusammensetzung einerseits und die nachgiebigen, hydrophilen, klassierten Feststoffteilchen andererseits in einem Gewichtsverhältnis von zwischen 2 und 100 zugesetzt sind.

15. Zement nach dem vorangehenden Anspruch,
**dadurch gekennzeichnet,**
**dass** das Pulver aus Polymethylmethacrylat-Vorpolymer, das Methylmethacrylat-Monomer und die strahlenundurchlässige Zusammensetzung einerseits und die nachgiebigen, hydrophilen, klassierten Feststoffteilchen andererseits in einem Gewichtsverhältnis zwischen 4 und 9 zugesetzt sind.
